# EUROPEAN PATENT APPLICATION

(11) **EP 2 399 525 A1**
(43) Date of publication of application: **28.12.2011**
(21) Application number: 10743837.6
(22) Date of filing: 19.02.2010
(51) Int. Cl.: A61B 17/00, A61B 17/22

(54) **INSTRUMENT FOR CAPTURING THROMBUS IN BLOOD VESSEL**

(30) Priority: 20.02.2009 JP 2009038326
(71) Applicant: Wellfind Co., Ltd., Saitama 343-0044 (JP)
(72) Inventor: SAKAI, Nobuyuki, Kobe-shi Hyogo 650-0046 (JP)
(74) Representative: Henseler, Daniela
(86) International application number: PCT/JP2010/052538
(87) International publication number: WO 2010/095712

(57) **Abstract**

An intravascular thrombus capturing instrument comprising a wire body (10) inserted into a catheter (C) and retractable from the tip of the catheter, and a thrombus capturing part (20) fitted at the tip of the wire body, wherein the thrombus capturing part (20) includes a fixed section (21) fitted at the tip of the wire body (10), a stopper (22) secured to the wire body (10) at prescribed intervals from the fixed section, a slidable section (23) slidably provided around the wire body (10) posterior to the stopper, and an elastic coil (24) bridging between the fixed section (21) and the slidable section (23). This securely captures and removes embolus such as thrombus (T) clung to a vascular wall.

## Description

### Technical Field

The present invention relates to a thrombus capturing instrument adapted to capture and remove thrombus formed in a blood vessel which causes cerebral infarction and cardiac infarction.

### Background Art

Conventionally, as therapy for thrombosis, it is known to the public mechanical therapy using medical equipments such as a catheter, in addition to chemotherapy administering a thrombolytic agent.
For example, the following Patent Document 1 discloses a mechanical blood clot treatment device, in which a core wire and a cable assembly are held in a sheath (catheter). In Patent Document 1, the sheath is advanced by crawling it in a blood vessel to a lesion where a thrombus is formed and the sheath is developed into coil shape by extruding a cage assembly from the tip of the sheath. Then, the thrombus clung to a vascular wall is mechanically removed by pulling out the cage assembly therefrom.

The following Patent Document 2 discloses an intravascular foreign-substance removing wire composed of a long wire body, a coiled foreign-substance capturing part fitted at the tip side of the wire body, and a manipulation wire.
In Patent Document 2, as with Patent Document 1, the wire body is advanced with a catheter to a lesion in a blood vessel where a foreign substance exists, and the substance is captured and removed with the foreign-substance capturing part fitted at the tip of the wire body and the manipulation wire.

The following Patent Document 3 discloses an embolectomy device with a long shaft having a base end and a leading end. In Patent Document 3 , as with Patent Document 1, a wire body is advanced with a catheter to a lesion in a blood vessel where a clot is formed, a strut at the tip of the wire body is expanded in a three dimensional direction by letting out the strut from the tip of the wire body, and the clot is extirpated using the strut.

### Prior Art Documents

### Patent Documents

Patent Document 1: JP 2003-33359 A
Patent Document 2: JP 2006-87630 A
Patent Document 3: JP 2006-516512 A

### Summary of the Invention

### Problem To Be Solved By the Invention

Meanwhile, these conventional thrombectomy tools have each been designed to expand a metal wire in coil shape or in the three dimensional direction to entrap the thrombus by the metal wire in an entangling manner or in a scrapping out manner to remove thereby the thrombus.
Such geometry so formed as above has, however, inexpediency that the metal wire is crushed in a pulling out direction or in an extruding direction when entrapping the thrombus in an entangling manner by the wire being expanded in coil shape or in the three dimensional direction. Accordingly, one encounters a difficulty in effectively capturing embolus such as thrombus clung to the vascular wall. What is worse, since these thrombectomy tools have the relatively complex structure, they might cause an unexpected trouble that a desired manipulation could not be carried out.
The present invention is made to solve such drawbacks and its objective is to provide a novel intravascular thrombus capturing instrument capable of securely capturing and removing the embolus such as the thrombus clung to the vascular wall.

### Solution to the Problem

To solve the drawbacks discussed above, a first aspect of an invention of an intravenous thrombus capturing instrument comprises a catheter advancing in a blood vessel, a wire body inserted into the catheter and retractable from the head of the catheter, and a thrombus capturing part fitted at the tip side of the wire body, wherein the thrombus capturing part includes a fixed section provided at the tip of the wire body, a slidable section slidably provided around the wire body posterior to the fixed section, an elastic coil bridging between the slidable section and the fixed p section, and a stopper fixed to the wire body located between the slidable section and the fixed section to restrict sliding of the slidable section.

Further, a second aspect of an invention is the intravenous thrombus capturing instrument according to the first aspect of an invention, wherein the elastic coil may be made by bridging one or plural superelastic hairlines urged in coil shape, with the wire body as an axis, between the fixed section and the slidable section.
Moreover, a third aspect of an invention is the intravenous thrombus capturing instrument according to the first or second aspect of an invention, wherein the elastic coil is urged in a shape diametrically reducing toward the tip of the wire body.

### Advantageous Effect of the Invention

According to the first invention, upon extruding the wire body from the tip of the catheter inserted into the blood vessel, the thrombus capturing part at the tip of the wire body is exposed and the elastic coil comes out consequently.
At this moment, the slidable section to which one end of the elastic coil is connected discontinues sliding anymore to the tip of the wire body viz. to the fixed section side by dint of the stopper. Hence, even if a force is applied to the elastic coil toward the fixed section of the tip of the wire body, the elastic coil may preserve its designated coil shape without being crushed.
This securely entraps the embolus such as the thrombus clung to the vascular wall in an entangling manner with the elastic coli, thus capturing and removing the thrombus thereby.

According to the second invention, the elastic coil is made by bridging one or plural hyperelastic hairlines urged in coil shape, with the wire body as an axis, between the fixed section and the slidable section. Thus, the elastic body may be easily held in the catheter as well as be deformed into the designated coil shape by dint of an elastic force potentially it has after extrusion from the tip of the catheter. This allows the elastic coil to easily gain access to a lesion in the blood vessel where objective thrombus is formed, and, after extrusion, to securely entrap in an entangling manner and remove the embolus such as thrombus clung to the vascular wall.

According to the third invention, since the elastic coil is urged so as to diametrically reduce toward the tip of the wire body, the invention may provide a coil shape having a large diameter without unduly prolonging the length of the hyperelastic hairlines. This reduces the sliding amount of the slidable section as well an overhang of the wire body, thereby surely developing the wire body into coil shape after extrusion thereof.

### Brief Description of the Drawings

FIG. 1A is a side view illustrating a state where the intravascular thrombus capturing instrument 100 according to the present invention is extruded from the tip of the catheter C, and FIG. 1B is a side view illustrating a state where the slidable section 23 is slid in a direction away from the stopper 22;
FIG. 2 is a side sectional view illustrating a state where the intravascular thrombus capturing instrument 100 according to the present invention is held in the catheter C;
FIG. 3 is a conceptual diagram illustrating a state where the intravascular thrombus capturing instrument 100 according to the present invention advanced in the blood vessel B;
FIG.4 is a conceptual diagram illustrating a state where the wire body 10 is extruded toward the thrombus T from the state shown in FIG. 3;
FIG. 5 is a conceptual diagram illustrating a state where the wire body 10 is further extruded toward the thrombus T from the state shown in FIG. 4;
FIG. 6 is a conceptual diagram illustrating a state where the hyperelastic hairlines W are released urging from the state shown in FIG. 5 and developed into the elastic coil 24;
FIG. 7 is a conceptual diagram illustrating a state where the wire body 10 is pulled out from the state shown in FIG. 5 and the thrombus T is entrapped in an entangling manner with the elastic coil 24;
FIG. 8 is a conceptual diagram illustrating a state where thrombus T entrapped in an entangling manner in the sate shown in FIG. 7 is captured and removed as it is with the elastic coil 24; and
FIG. 9 is a conceptual diagram illustrating a state where of the blood vessel B from which almost all the thrombus T is removed.

### Description of Embodiments

A description will be made to the embodiment of the present invention with reference to the accompanying drawings.

### (Configuration)

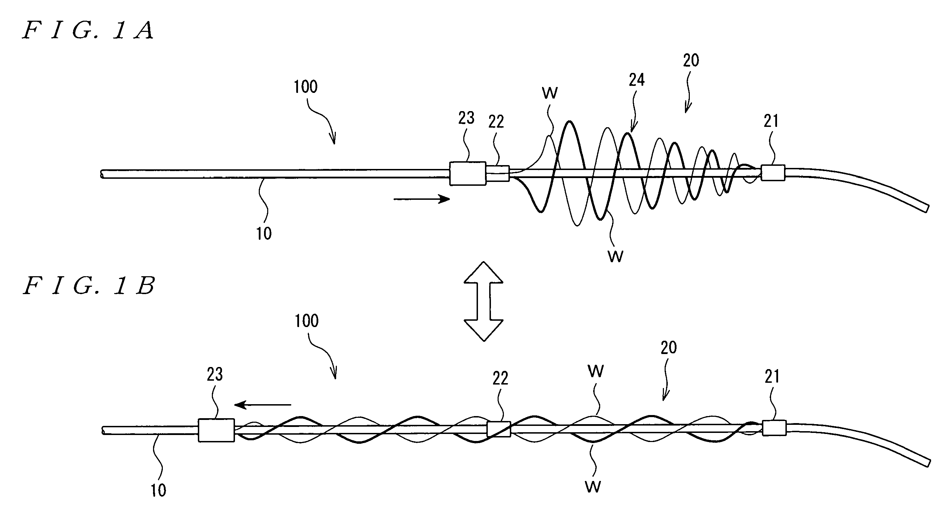

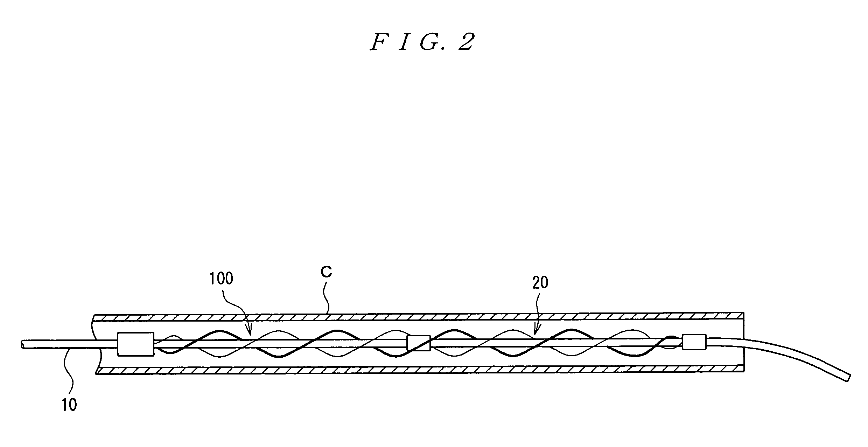

FIG. 1 and FIG. 2 are drawings illustrating one embodiment of the intravascular thrombus capturing instrument 100 according to the present invention.
As shown in FIGS, the thrombus capturing instrument 100 mainly comprises a wire body 10 inserted into a micro catheter C of which tip is opened, and a thrombus capturing section 20 fitted at the tip of the wire body 10.

The thrombus capturing section 20 comprises a fixed section 21 provided at the tip of the wire body 10, a stopper 22 secured to the wire body 10 at prescribed intervals from the fixed section 21, a slidable section 23 slidably provided around the wire body 10 posterior to the stopper 22, and an elastic coil 24 bridging between the slidable section 23 and the fixed section 21.
As shown in FIG. 2A, the elastic coil 24 is made by bridging a pair of hyperelastic hairlines W,W urged in coil shape, with the wire body 10 as an axis, between the fixed section 21 and the slidable section 23.

The hyperelastic hairlines W are made up e.g. of hyperelastic alloy etc. such as nickel-titanium alloy which has no adverse impact on a living body, of which diameter is e.g. 0.03 mm or so. In the embodiment, the pair of hyperelastic hairlines W,W each form a left-handed coil and a right-handed coil and construct an elastic coil 24 in such a manner as to overlap their axis each other on a straight line. While in the embodiment, the elastic coil 23 is made of the pair of the hyperelastic hairlines W, W, they may of course be made of one, or three or more hyperelastic hairlines W.

In the meantime, the fixed section 21 and the stopper 22 are integrally secured so as to diametrically expanding the wire body 10. Further, the slidable section 23 is made of a metal tube, which is slidable along the wire body 10 but its further sliding to the fixed section 21 side is restricted by interfering with the stopper 22. Thus, as shown in FIG. 1B, when the slidable section 23 is slid so as to part from the stopper 22, the hyperelastic hairlines W constructing the elastic coil 24 prolong and deform so as to closely contact the wire body 10. Incidentally, the wire body 10, the fixed section 21, the stopper 22, and the slidable section 23 may also be made of nickel-titanium alloy in the exact same way as the hyperelastic hairlines W. Additionally, they are made of materials such as stainless steel (SUS) or synthetic resins which have no adverse impact on a living body.

### (Operation)

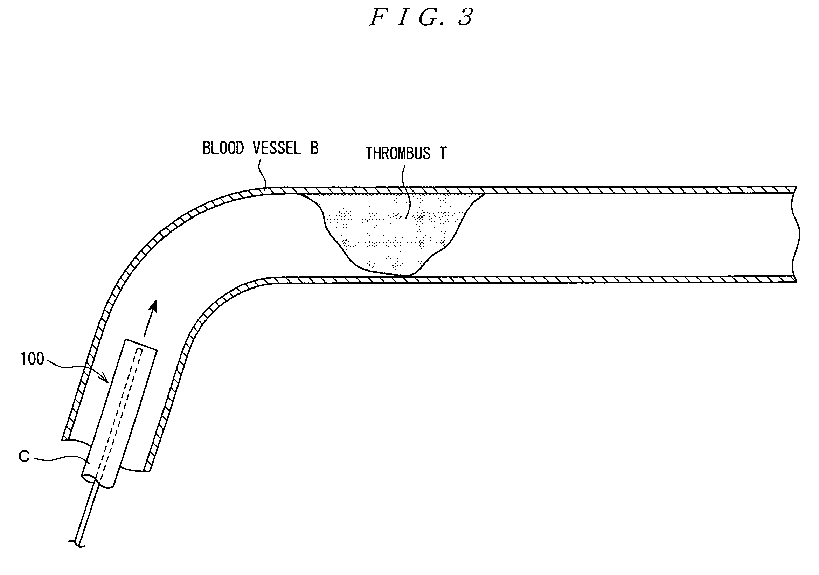

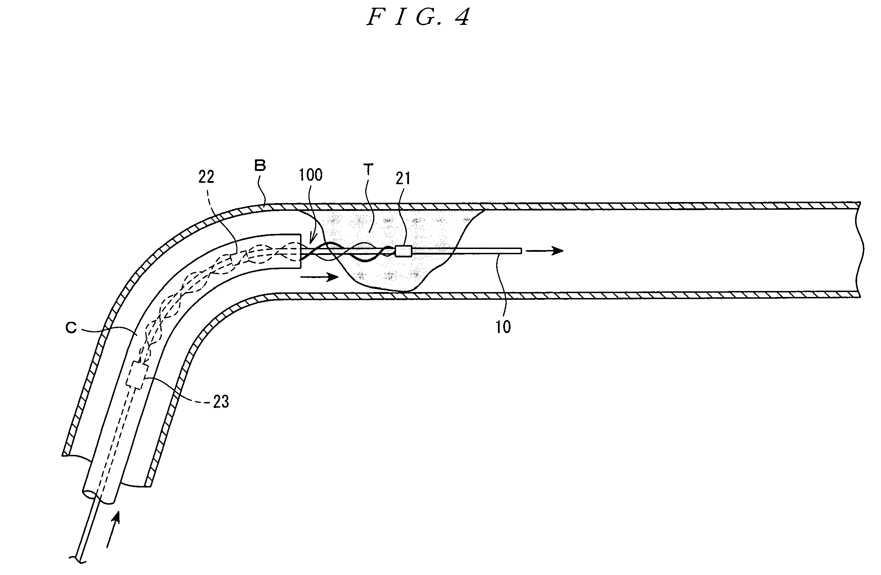

An explanation will next be made, referring to FIG. 3 to FIG. 9, to an operation exerted by the intravascular thrombus capturing instrument 100 having such configuration according to the present invention, namely a removal process of the thrombus clung to the vascular wall.
The tip of the wire body 10 is introduced in the blood vessel B with the tip of the wire body 10 held in the catheter C, and positions the head of the catheter C in the vicinity of the target thrombus T, as shown in FIG. 3.
The wire body 10 is then extruded from the head of the catheter C as shown in FIG. 4. At this time, because the thrombus T is generally in a jelly-like relatively soft state, the tip of the wire body 10 pushes its way just as it is through the thrombus T and is forwarded ahead of the thrombus T.

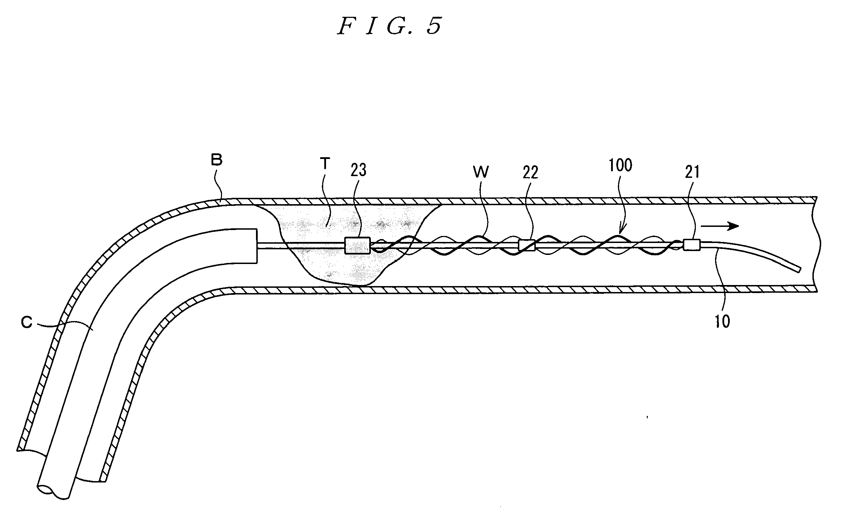

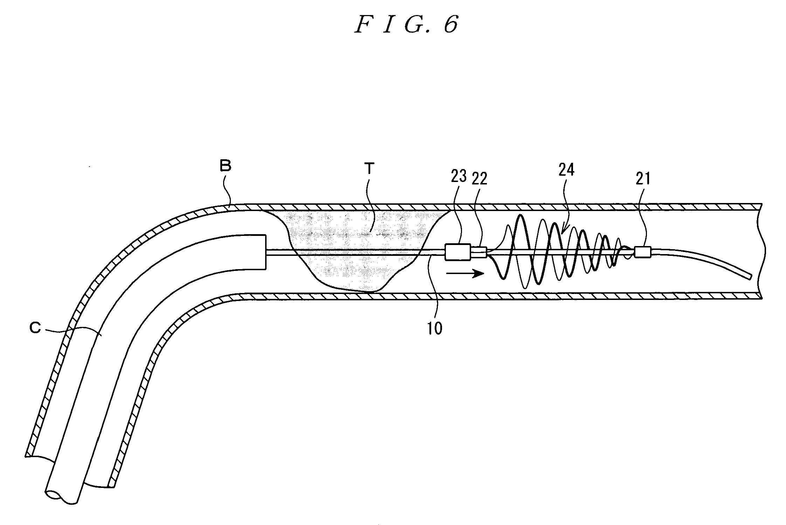

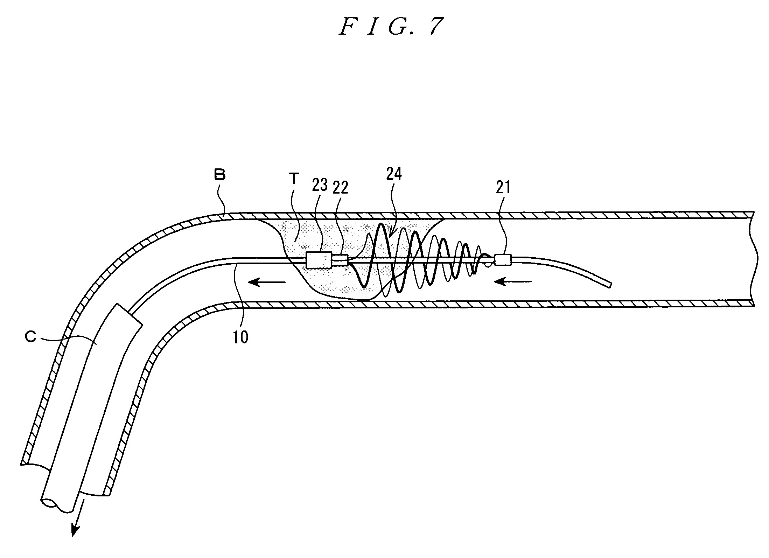

Subsequently, as shown in FIG. 5 and FIG. 6, when the wire body 10 further pushes its way through the thrombus T and the most of the elastic coil 23 fitted at the tip thereof pushes its way through the thrombus T, the hyperelastic hairlines W of which urging is released to deform in coil shape to be developed into the elastic coil 24.
The moment such elastic coil 24 is developed, pulling out the wire body 10 in an opposite direction, the elastic coil 24 irrupts into the thrombus T and acts so that the thrombus T is entrapped in an entangling manner from the vascular wall, as shown in FIG. 7.
At this juncture, the slidable section 23 one end of which is connected to the elastic coil 24 discontinues sliding anymore to the tip of the wire body 10, namely to the fixed section 21 side by dint of the stopper 22. Thus, the elastic coil 24 may preserve its prescribed coil shape, without being crushed, even if a force is applied to the elastic coil 24 toward the fixed section 21 of the wire body 10, due to the resistance of the thrombus T.

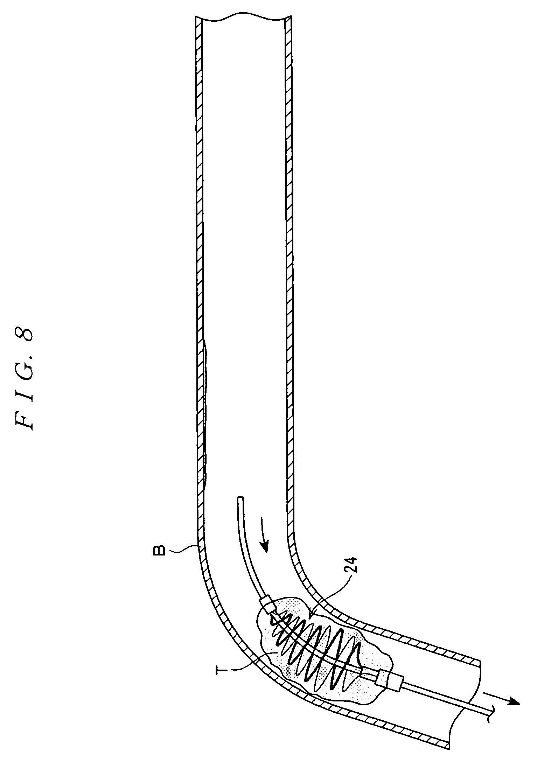

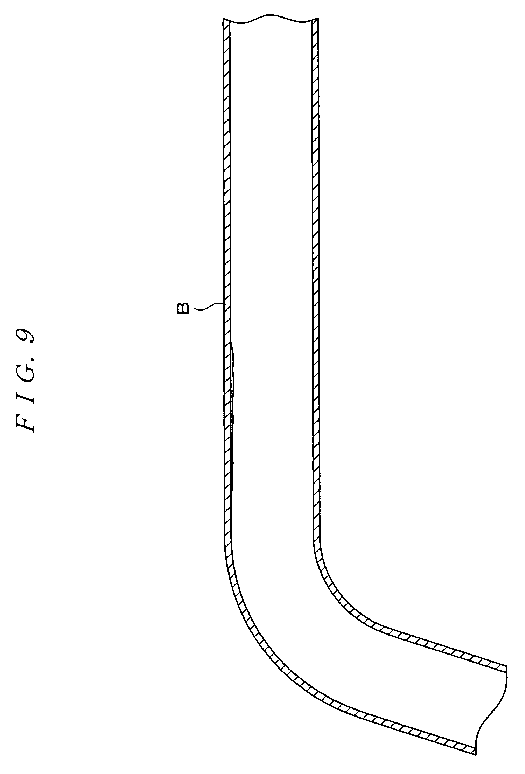

Accordingly, as shown in FIG. 8 and FIG. 9, further pulling out the wire body 10 together with the catheter C in this state entraps almost all the thrombus T in entangling manner all over the elastic coil 24 as it is, and removes the thrombus T from the interior of the blood vessel B.
Namely, assume that in the absence of the stopper 22 between the fixed section 21 and the slidable section 23, it follows that the slidable section 23 further slides to the fixed section 21 side due to the resistance of the thrombus T, impeding the elastic coil from developing a predetermined coil shape. As a result, it ends up in causing the disadvantage that it fails to entrap in an entangling manner and capture radically the thrombus T clung to the blood vessel.

By contrast, in the thrombus capturing instrument 100 of the present embodiment, the slidable section 23 discontinues sliding anymore to the tip of the wire body 10, namely to the fixed section 21 side by dint of the stopper 22. Thus, the elastic coil may preserve the prescribed coil shape, without being crushed, even if a force is applied toward the fixed section 21 at the tip of the wire body 10.
This securely entraps in an entangling manner the thrombus T clung to the blood wall and removes the thrombus T as it is with the coil 24. The entrapped thrombus T is dragged as it stands into a guiding catheter (not shown) along with the thrombus capturing instrument 100 for taking out the thrombus T outside the body from the blood wall B together with the guiding catheter.

The following Table 1 indicates experimental results to evaluate the thrombus capturing effects with the thrombus capturing instrument 100 of the present invention having such configuration.
More precisely, an experiment is conducted for capturing coffer jelly by filling the coffer jelly by 3 cm in width in a silicon tube of internal diameter of 4 mm, and by inserting the thrombus capturing instrument 100 of the present embodiment into the silicon tube, presuming the silicon tube to be the blood vessel and the coffer jelly to be the thrombus.
In the experiment, three types of the elastic coil 24 are used for conducting the experiment 10 times for each: a coil made of one superelastic hairline W (single spiral), a coil made of two superelastic hairlines W (double spiral), and a coil with no stopper 22.

| Thrombus Capturing Amount (mg) | Single Spiral | Double Spiral | No stopper |
|---|---|---|---|
| 1st | 10 | 65 | Removal succeeded but capture failed |
| 2nd | 10 | 63 | |
| 3rd | 13 | 71 | |
| 4th | 14 | 73 | |
| 5th | 10 | 64 | |
| 6th | 11 | 70 | |
| 7th | 15 | 56 | |
| 8th | 12 | 65 | |
| 9th | 14 | 71 | |
| 10th | 10 | 64 | |
| Mean Value | 11.9 | 67.2 | |

Consequently, as is apparent from Table 1, in both cases where the elastic coil may have the single spiral and the double spiral structures, it was possible to capture the coffer jelly filled in the silicon tube. Additively, the elastic coil having the double spiral structure may exert an excellent capturing performance approximately five times higher than exerted by the elastic coil having the single spiral.
By contrast, in the case of the elastic coil 24 without the stopper 22, while it just barely succeeded to remove the coffer jelly filled in the silicon tube to a certain amount, it failed to capture the removed coffer jelly as it was with the elastic coil 24 for lack of preserbavility of the designated coil shape thereof.
According to the thrombus capturing instrument 100, since the elastic coil 24 may preserve the designated coil shape, without being crushed, even if a force is applied to the coiled body 24, the instrument 100 enables securely entrapping the thrombus T clung to the blood wall in an entangling manner and removing it with the elastic coil 24, thus capturing and removing the thrombus T therewith.

Further, the elastic coil 24 is made by bridging one or plural superelastic hairlines W urged in coil shape, with the wire body 10 as an axis, between the fixed section 21 and the slidable section 23. Hence, the body can be easily held in the catheter C, as shown in FIG. 1, as well as it can be deformed in a prescribed coil shape by dint of its elastic force after extrusion from the catheter C, as shown in FIG. 2. This allows the elastic coil 24 to easily gain access up to a lesion in the blood vessel B where the target thrombus T is formed as well as the thrombus T clung to the blood vessel to surely entrap in an entangling manner and capture the thrombus T, after being got to the target thrombus T.

Furthermore, taking the geometry where the elastic coil 24 is urged so as to diametrically reduce toward the tip of the wire body 10, as shown in FIG. 2, may provide a coil shape having a large diameter without unnecessarily prolonging the length of the superelastic hairlines W.
This reduces the slide amount of the slidable section 23 which reduces an overhang of the wire body 10 as well as ensures establishment of the coil shape after extrusion thereof.

Besides, since the thrombus capturing instrument 100 of the present invention has the relatively simpler structure than the conventional ones, it is free from the occurrence of an unexpected trouble that a desired manipulation could not.
Alternatively, as a variation of the present invention, a second thrombus capturing part smaller just one size, may further be provided between the fixed section 21 and the stopper 22 to implement a dual structure, in addition to the above configuration.

### Industrial Applicability

The present invention enables, in the medical field, surely capturing and removing the thrombus formed in the blood vessel, which may cause thrombosis such as cerebral infarction and cardinal infarction.

### Reference Signs List

100: thrombus capturing instrument; 10: wire body; 21: fixed section; 22: stopper; 23: slidable section; B: blood vessel; C: catheter; T: thrombus; W: superelastic hairlines.

## Claims

1. An intravenous thrombus capturing instrument, comprising:
a catheter advancing in a blood vessel;
a wire body inserted into the catheter and retractable from the head of the catheter; and
a thrombus capturing part fitted at the tip side of the wire body,
wherein the thrombus capturing part includes:
a fixed section provided at the tip of the wire body;
a slidable section slidably provided around the wire body posterior to the fixed section;
an elastic coil bridging between the slidable section and the fixed section; and
a stopper fixed to the wire body located between the slidable section and the fixed section to restrict sliding of the slidable section.

2. The intravenous thrombus capturing instrument according to claim 1, wherein the elastic coil is made by bridging one or plural superelastic hairlines urged in coil shape, with the wire body as an axis, between the fixed section and the slidable section.

3. The intravenous thrombus capturing instrument according to claim 1 or claim 2, wherein the elastic coil is urged in a shape diametrically reducing toward the tip of the wire body.
